# EUROPÄISCHE PATENTSCHRIFT

(11) **EP 0 510 343 B1**
(45) Veröffentlichungstag und Bekanntmachung des Hinweises auf die Patenterteilung: **21.06.1995**
(21) Anmeldenummer: 92104253.7
(22) Anmeldetag: 12.03.1992
(51) Int. Cl.: A61F 5/44

(54) **Halterung für einen Urinbeutel**
Urine bag harness
Point d'attache de sac destine à recueillir les urines

(30) Priorität: 22.04.1991 DE 4113133
(43) Veröffentlichungstag der Anmeldung: 28.10.1992
(73) Patentinhaber: Müller, Karl Heinz, D-51674 Wiehl (DE); Müller, Klaus, D-51674 Wiehl (DE); Müller, Peter, D-51674 Wiehl (DE)
(72) Erfinder: Selzer, Klaus, W-5276 Wiehl (DE)
(74) Vertreter: Neumann, Ernst Dieter, Dipl.-Ing.

(56) Entgegenhaltungen:
- CH-A- 662 725
- DE-A- 2 917 583
- US-A- 3 648 699

## Beschreibung

Die Erfindung betrifft eine Halterung für einen Urinbeutel am Bein, insbesondere am Oberschenkel eines Benutzers. Derartige Urinbeutel bestehen aus zweilagigem Kunststoffmaterial, das an den Rändern rundum verschweißt ist und oben einen Zulauf mit einem Aufsteckkonus für den Ableiter eines Kondomurinales und unten einen Ablauf mit einer verschließbaren Ventilöffnung haben. Jeweils am oberen und unteren verschweißten Randbereich sind Öffnungen oder Ösen vorgesehen, durch die Velcrobänder (Klettbänder) zur Befestigung durchgezogen werden können, die um den Oberschenkel herumgelegt und mit ihren Enden aneinander befestigt werden können. Derartige Bänder schneiden sich in unangenehmer Weise im Oberschenkel ein. Bänder gleicher Art können auch um die Hüfte des Benutzers gelegt und dort verschlossen werden, wobei ein angehängter Urinbeutel über Laschen oder dergleichen wiederum in Höhe des Oberschenkels gehalten wird. Das Problem des Einschnürens ist hierbei das gleiche. In beiden Fällen ist die Befestigung nicht nur unbequem, sondern birgt auch die Gefahr einer Störung der ungehinderten Durchblutung. Urinbeutel und Halterungen der vorstehenden Art sind in der DE 32 41 517 C1 beschrieben.

Aus der CH 662 725 A5 ist eine Halterung für Urinbeutel der vorstehend genannten Art bekannt, die die Form von kurzen Unterhosen mit am Beinaustritt angenähten, den Oberschenkel bedeckenden Lappen hat. Aus der gleichen Schrift ist eine Halterung dieser Art bekannt, die die Form von Unterhosen mit zumindest knielangen Beinen aufweist. Hierbei sind in beiden Fällen nur Befestigungslöcher für den Urinbeutel im Bereich des auf dem Oberschenkel liegenden Materials vorgesehen, an denen dieser befestigt werden kann. Zur Befestigung sollen Knöpfe oder die bereits genannnten Velcrobänder dienen. Der Urinbeutel wird dabei in nachteiliger Weise unterhalb des Materials getragen, so daß ein direkter Hautkontakt zustande kommt. Die Halterungen stellen Konfektionsware dar, an der nach der Herstellung noch zusätzliche Bearbeitungsschritte erforderlich sind.

Aus der DE 29 17 583 A1 ist eine Halterung der genannten Art bekannt, die ein einzelnes bis zur Hüfte reichendes Hosenbein aufweist, das mit einem durch eine Schlaufe gezogenen Taillenband am Benutzer festgehalten wird. Auf das genannte Hosenbein ist eine Tasche aufgenäht, in die ein Urinbeutel der genannten Art eingesteckt wird. Auch hierbei handelt es sich wieder um ein konfektioniertes, d. h. aus mehreren Teilen zugeschnittenes und miteinander vernähtes Teil, dessen Fertigung aufwendig ist, und das nicht als billiges Verbrauchsmaterial zum relativ kurzzeitigen Gebrauch in Betracht kommt.

Aus der US 3 648 699 ist eine hosenförmige Halterung für saugfähige Tucheinlagen bekannt, die jedoch nicht für die Halterung von Urinbeuteln der obengenannten Art vorgesehen ist.

Aus einem Prospekt der Firma Pruban, Tschechoslowakei, ist endlos gewirktes elastisches Schlauchmaterial für Verbandszwecke bekannt, über dessen Gebrauch offenbart ist, daß das Schlauchmaterial bei Anwendung an Kopf und Gliedmaßen doppelt gelegt werden kann. An den Körperteilen zu fixierende Verbandspakete liegen hierbei unmittelbar auf der Haut des Patienten unterhalb der zumindest einen Lage des Schlauchmaterials. An besonders exponierten Stellen, z. B. der Ferse am Fuß verbundenen Patienten, können in das Schlauchmaterial nachträglich Löcher eingeschnitten werden.

Hiervon ausgehend ist es Aufgabe der vorliegenden Erfindung, eine Halterung für Urinbeutel am Bein, insbesondere am Oberschenkel des Benutzers bereitzustellen, die einfach und billig herstellbar ist und den Tragekomfort verbessert. Die Lösung hierfür ist gekennzeichnet durch einen ersten Wirkschlauchteil, der über das Bein ziehbar ist, einen zweiten Wirkschlauchteil gleichen Durchmessers, der über einen umlaufenden Verbindungsbereich mit dem ersten verbunden ist und zur Fixierung des Urinbeutels auf den ersten Wirkschlauchteil von unten nach oben umschlagbar ist, sowie eine Trennstelle im Verbindungsbereich zum Durchtritt eines Ablaufes des Urinbeutels bei übereinandergeschlagenen Wirkschlauchteilen. Eine derartige Halterung aus Wirkschlauch ist äußerst billig herstellbar, erspart das mühselige Anbringen von Verbindungsmitteln am Urinbeutel, insbesondere das Durchziehen von Bändern durch die Ösen im Randbereich und hat darüber hinaus den Vorteil, die elastischen Haltekräfte auf eine große Fläche zu verteilen, so daß die Gefahr einer Behinderung der Durchblutung wesentlich reduziert ist. Der erfindungsgemäße Wirkschlauch wird mit beiden aneinander anschließenden Teilen über das Bein gezogen, der Urinbeutel lose auf den oberen Wirkschlauchteil aufgelegt und der untere Wirkschlauchteil über den oberen Wirkschlauchteil unter Einschluß des dazwischen zu liegen kommenden Urinbeutels umgeschlagen, wobei das Ablaufventil durch die Trennstelle im Verbindungsbereich durchgesteckt wird. In günstiger Weise ist hiermit ein unmittelbarer Hautkontakt des Kunststoffes auf dem Bein, der zu Unannehmlichkeiten beim Tragen führen könnte, verhindert.

Um die elastische Spannung der Wirkschlauchteile auf das Bein, insbesondere den Oberschenkel möglichst weitgehend zu reduzieren und den Durchfluß durch den Urinbeutel nicht zu behindern, ist vorzugsweise nur der erste obere Wirkschlauchteil mit einem größeren Anteil elastischer Fäden gewirkt. Dieser Teil bewirkt die eigentliche Halterung auf dem Oberschenkel. Dagegen ist der untere, außen zu liegen kommende Wirkschlauchteil bevorzugt mit weniger elastischen Fäden oder unter Verzicht auf elastische Fäden hergestellt, da er nur das Umklappen oder Verrutschen des Urinbeutels verhindern muß, nicht jedoch dessen Gewicht über Reibungskräfte aufnehmen muß.

Zur Sicherung gegen das vorhergenannte Umklappen des unteren, außen auf dem Urinbeutel zu liegen kommenden Schlauchteils kann dieser mit einem abschließenden Bündchen mit erhöhter elastischer Spannung versehen sein.

Ergänzend oder alternativ hierzu kann der obere erste Wirkschlauchteil, der unmittelbar auf dem Oberschenkel zu liegen kommt, zur Erhöhung der Reibwirkung unter Verwendung von gummierten Fäden hergestellt sein, so daß hierdurch ebenfalls die notwendige elastische Vorspannung der Wirkschlauchteile auf den Oberschenkel reduziert werden kann.

Wird der Urinbeutel in grundsätzlich gleicher Weise wie vorher beschrieben auf dem Unterschenkel befestigt, ist es günstig, zumindest im ersten Wirkschlauchteil, gegebenenfalls auch zusätzlich im zweiten Wirkschlauchteil, jeweils einen Schlitz vorzusehen, der mit der Kniescheibe des Benutzers zur Deckung gebracht wird. Durch ein derartiges Anhängen können zusätzliche Haltekräfte aufgebracht werden.

Nach einer zweiten günstigen Ausgestaltung kann der obere Wirkschlauchteil ein Beinteil einer Hose bilden, wobei diese Hose dann bevorzugt zwei ungleich lange Beinteile aufweisen kann. In einer derartigen Ausgestaltung wird das Gewicht des im übrigen von oberem Wirkschlauchteil und unterem Wirkschlauchteil in gleicher Weise gehaltenen Urinbeutels über die Hosenoberfläche aufgenommen. Es kann hierbei auf die Verwendung eines größeren elastischen Fadenanteiles im Bereich des oberen Wirkschlauchteils verzichtet werden. Eine derartige Hose kann so gestaltet werden, daß sie nur ein einzelnes besonders ausgebildetes Beinteil umfaßt, das aus dem ersten oberen Wirkschlauchteil gebildet wird, auf dem der Urinbeutel zu liegen kommt und über das der zweite untere Wirkschlauchteil übergeschlagen wird, während es möglich ist, das zweite Beinteil sehr viel kürzer auszubilden. Es ist jedoch auch möglich, das zweite Beinteil genauso lang wie die beiden Wirkschlauchteile im übereinandergeschlagenen Zustand zu machen.

Zum erleichterten Auffinden der Trennstelle für den Durchtritt des Ablaufes ist es sinnvoll, den Verbindungsbereich zwischen den beiden Wirkschlauchteilen mit zumindest einem farblich abgesetzten Markierungsfaden herzustellen, so daß die Trennstelle zum Durchstecken des Ablaufventils leichter auffindbar ist. Im übrigen kann der Verbindungsbereich aus lose gewirkten Fäden bestehen, die das Umschlagen an dieser Stelle erleichtern.

Der Erfindung liegt weiterhin die Aufgabe zugrunde, ein besonders einfaches und kostengünstiges Verfahren zur Herstellung der erfindungsgemäßen Halterungen herzustellen. Die Lösung besteht bei den Halterungen gemäß der ersten Ausführung darin, daß die Wirkschlauchteile fortlaufend in zwei Lagen (in doppelfonturiger Wirkweise) quer zur Längsrichtung der Wirkschlauchteile gewirkt werden, indem in gleichmäßigen Abständen die beiden Lagen zur Bildung von Naht- und Trennbereichen über die gesamte Breite der Lagen ineinandergewirkt werden, wobei jeweils in einer der beiden Lagen zwischen den beiden Wirkschlauchteilen quer zur Längsrichtung der Wirkschlauchteile eine Trennstelle vorgesehen ist und daß durch mittiges Trennen längs der genannten Naht- und Trennbereiche einzelne Halterungen fertiggestellt werden. Durch das erfindungsgemäße Verfahren stehen mit dem Abtrennen einzelner Wirkschlauchteile unmittelbar fertige erfindungsgemäße Halterungen zur Verfügung, die in keiner Weise mehr bearbeitet werden müssen, so daß der Lohnanteil bei der Fertigung äußerst gering ist. Es ist auch möglich, anstelle der hier beschriebenen Trennung eine Vortrennung vorzunehmen, die das Verpacken der noch zusammenhängenden Wirkschlauchteile erleichtert und ein fertiges Abtrennen durch Abreißen beim Verbraucher ermöglicht.

Ein Verfahren zur Herstellung von Halterungen gemäß der zweiten Ausführungsform besteht darin, daß Hosen fortlaufend und paarweise in zwei Lagen (in doppelfonturiger Wirkweise) quer zur Längsrichtung der Beinteile gewirkt werden, indem in gleichmäßigen Abständen die beiden Lagen zur Bildung von Naht- und Trennbereichen ineinandergewirkt werden, wobei jeder zweite Naht- und Trennbereich über die gesamte Breite der Lagen verläuft und jeder zwischen diesen liegende Naht- und Trennbereich parallel dazu in gleichem Abstand von den ersteren nur einen mittleren Teilbereich symmetrisch zu den Seitenkanten der Lagen umfaßt und wobei von den Enden der letzteren Naht- und Trennbereiche senkrecht zu den Beinteilen verlaufende, bis zu den ersteren Naht- und Trennbereichen reichende deckungsgleiche Trennschlitze in beiden Lagen verlaufen, wobei jeweils in einer der Lagen senkrecht zur Richtung der Beinteile eine weitere kurze Trennstelle vorgesehen ist, und daß durch mittiges Trennen der genannten Naht- und Trennbereiche einzelne Halterungen in Form von Hosen voneinander abgetrennt werden. Auch das hiermit beschriebene Verfahren führt zur fertigen Halterung an der keine weiteren Nacharbeiten mehr erforderlich sind und die durch die fortlaufende Wirktechnik besonders kostengünstig herzustellen sind.

Bevorzugte Ausführungsbeispiele der Erfindung sind in den Zeichnungen dargestellt.
- Fig. 1: zeigt Halterungen in einer ersten Ausführung bei der Herstellung;
- Fig. 2: zeigt eine Halterung in der Ausführung nach Fig. 1 in Benutzung in zwei Stadien;
- Fig. 3: zeigt Halterungen in einer zweiten Ausführung in zwei Varianten;
- Fig. 4: zeigt eine Halterung gemäß Fig. 3 in der Anwendung.

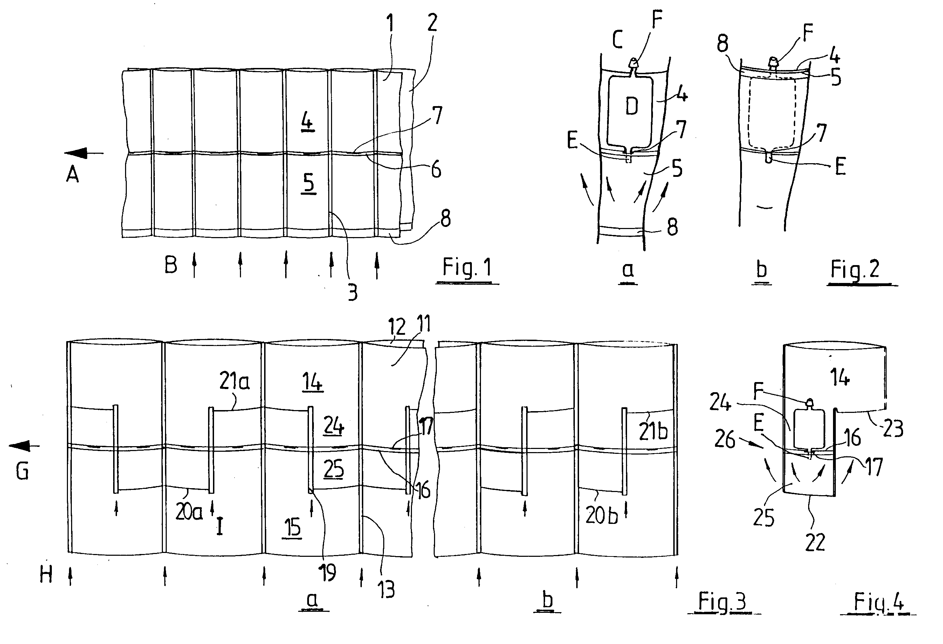

In Figur 1 sind eine obere Lage 1 und eine untere Lage 2 einer doppelfonturigen Wirkbahn gezeigt, die in Abständen entlang von quer zur durch den Pfeil A angedeuteten Produktionsrichtung entlang von Trenn- und Wirkbereichen 3 ineinandergewirkt sind. Bei einem Trennen entlang dieser Trenn- und Wirkbereiche 3 gemäß den Pfeilen B entstehen hieraus einzelne Schlauchprodukte, die einen ersten oberen Wirkschlauchteil 4 und einen zweiten unteren Wirkschlauchteil 5 umfassen. Der obere Schlauchbereich kann einen erhöhten Anteil elastischer Fäden umfassen. Zwischen beiden ist ein Verbindungsbereich 6 erkennbar, der in besonderer Art gewirkte Fäden umfassen kann. Zumindest umfaßt dieser Verbindungsbereich 6 in bevorzugter Weise zumindest einen farblich abgesetzten Markierungsfaden, anhand dessen sich die Position von ausschließlich in der oberen Lage 1 vorgesehenen Trennstellen 7 identifizieren läßt. Im Bereich des unteren Wirkschlauchteiles 5 sind entlang der Kanten Bündchenbereiche 8 mit einem erhöhten Anteil elastischer Fäden ausgebildet.

In Figur 2 ist eine aus oberem Wirkschlauchteil 4 und unterem Wirkschlauchteil 5 bestehende Halterung bis zum Oberschenkel über ein Bein C gezogen. Im Bereich des oberen Wirkschlauchteils 4 ist ein Urinbeutel D aufgelegt. Dessen unteres Abflußventil E ist bereits durch eine Trennstelle 7 durchgesteckt. Gemäß den in a) angedeuteten Pfeilen wird nun der untere zweite Wirkschlauchteil 5 über den oberen Wirkschlauchteil 4 und den Urinbeutel geschlagen, so daß - wie in b) gezeigt - dessen Aufsteckkonus F ebenso wie das Abflußventil E freiliegt und der Urinbeutel zwischen den Wirkschlauchteilen gehalten ist.

In Figur 3 sind eine obere Lage 11 und eine untere Lage 12 einer doppelfonturigen Wirkbahn an ersten Naht- und Trennstellen 13 über die gesamte Bahnbreite ineinandergewirkt, während jeweils zwischen Naht- und Trennstellen 13 je eine Naht- und Trennstelle 19 vorgesehen ist, die mit gleichem Abstand zu ersteren parallel zu diesen verläuft und nur einen Teil der Breite der Lagen symmetrisch zu den Seitenkanten liegend umfaßt. Die Produktionsrichtung der Bahn ist durch den Pfeil G angezeigt. Jeweils zwischen den Naht- und Trennstellen sind in Bahnrichtung verlaufende erste Trennschlitze 20a, 21a, 20b, 21 b vorgesehen, die in beiden Lagen 11 und 12 verlaufen. Es ist weiterhin ein vorzugsweise farblich abgesetzter Markierungsstreifen 16 in zumindest einer der Lagen vorgesehen, in dem jeweils kurze Trennschlitze 17 ebenfalls ausschließlich in einer der Lagen angeordnet sind. Durch Pfeile H, I ist angedeutet, daß längs der Naht- und Trennstellen eine mittige Trennung vorgesehen ist, die im Bereich der Trennstellen 19 beidendig nicht aus dem Nahtbereich herauslaufen darf. Die Figuren a) und b) unterscheiden sich in der Lage der Trennschlitze 20, 21 zwischen den einzelnen Trennstellen 13.

Durch die Lage der Trennstellen 13, 19 und die Lage der Trennschlitze 20, 21 sind jeweils zwischen zwei Trennstellen 13 bereits Hosen 14, 15 als Halbfertigprodukte erkennbar. An jeder der Hosen 14, 15 sind ein erster Wirkschlauchteil 24 und ein zweiter Wirkschlauchteil 25 erkennbar, zwischen denen jeweils eine Trennstelle 17 in nur einer der Lagen 11 liegt.

In Figur 4 ist eine Hose 14 dargestellt, die eine durch die Trennstelle 20 erzeugte erste Beinöffnung 22 und eine durch die Trennstelle 21 erzeugte zweite Beinöffnung 23 aufweist. Die Beinöffnung 22 bildet das untere Ende eines längeren Beinteils 26. Ein Urinbeutel D ist auf den oberen Abschnitt des Beinteils 26 aufgelegt, wobei dessen Abflußventil E bereits durch eine Trennstelle 17 im Markierungsbereich 16 hindurchgesteckt ist. Wie durch die Pfeile angedeutet, kann der untere Wirkschlauchteil 25 des Beinteils 26 nunmehr auf den oberen Wirkschlauchteil 24, ähnlich wie in Figur 2b übergeschlagen werden. Auf die Ausbildung und Darstellung eines Bündchenteiles ist hierbei verzichtet worden.

### Bezugzeichenliste

- 1: obere Lage
- 2: untere Lage
- 3: Naht- und Trennbereich
- 4: erster Wirkschlauchteil
- 5: zweiter Wirkschlauchteil
- 6: Verbindungsbereich
- 7: Trennstelle
- 8: Bündchen
- 9:
- 10:
- 11: obere Lage
- 12: untere Lage
- 13: Trennstelle
- 14: Hose
- 15: Hose
- 16: Markierungsbereich
- 17: Trennstelle
- 18:
- 19: Naht- und Trennbereich
- 20: Trennstelle
- 21: Trennstelle
- 22: Beinöffnung
- 23: Beinöffnung
- 24: Wirkschlauchteil
- 25: Wirkschlauchteil
- 26: Beinteil
- A: Herstellrichtung
- B: Trennrichtung
- C: Oberschenkel
- D: Urinbeutel
- E: Abflußstutzen
- F: Verbindungskonus
- G: Herstellrichtung
- H: Trennrichtung
- I: Trennrichtung

## Patentansprüche

1. Halterung für einen Urinbeutel (D) am Bein, insbesondere am Oberschenkel (C) eines Benutzers, mit einem Schlauchteil, der über das Bein ziehbar ist,
gekennzeichnet dadurch, daß der Schlauchteil aus zwei Teilen besteht,
einem ersten Wirkschlauchteil (4, 24), der über das Bein (C) ziehbar ist, und einem zweiten Wirkschlauchteil (5, 25) gleichen Durchmessers, der über einen umlaufenden Verbindungsbereich (6, 16) mit dem ersten verbunden ist und zur Fixierung des Urinbeutels (D) auf den ersten Wirkschlauchteil (4, 24) von unten nach oben umschlagbar ist, sowie einer Trennstelle (7, 17) im Verbindungsbereich (6, 16) zum Durchtritt eines Ablaufs (E) des Urinbeutels (D) bei übereinandergeschlagenen Wirkschlauchteilen (4, 5, 24, 25).

2. Halterung nach Anspruch 1,
dadurch gekennzeichnet,
daß der erste Wirkschlauchteil (4) ein Gewirk mit höherem elastischen Fadenanteil aufweist.

3. Halterung nach einem der Ansprüche 1 oder 2,
dadurch gekennzeichnet,
daß der erste Wirkschlauchteil (4) zur Erhöhung der Reibwirkung unter Verwendung von gummierten Fäden hergestellt ist.

4. Halterung nach einem der Ansprüche 1 bis 3,
dadurch gekennzeichnet,
daß zumindest der erste Wirkschlauchteil (4) einen Schlitz zum Fixieren an der Kniescheibe des Benutzers bei Anordnung des Urinbeutels am Unterschenkel des Benutzers aufweist.

5. Halterung nach Anspruch 1,
dadurch gekennzeichnet,
daß der erste Wirkschlauchteil (24) ein Beinteil (26) einer Hose (14) bildet.

6. Halterung nach einem der Ansprüche 1 bis 5,
dadurch gekennzeichnet,
daß der Verbindungsbereich (6, 16) aus lose gewirkten Fäden besteht.

7. Halterung nach einem der Ansprüche 1 bis 6,
dadurch gekennzeichnet,
daß im Verbindungsbereich (6, 16) zumindest ein farblich abgesetzter Markierungsfaden eingesetzt ist.

8. Halterung nach einem der Ansprüche 1 bis 7,
dadurch gekennzeichnet,
daß am freien Ende des zweiten Wirkschlauchteiles (5, 25) ein Haltebündchen (8) vorgesehen ist.

9. Verfahren zur Herstellung einer Halterung nach Anspruch 1,
dadurch gekennzeichnet,
daß die Wirkschlauchteile (4, 5) fortlaufend in zwei Lagen (1, 2) in doppelkonturiger Wirkweise quer zur Längsrichtung der Wirkschlauchteile gewirkt werden, indem in gleichmäßigen Abständen die beiden Lagen zur Bildung von Naht- und Trennbereichen (3) über die gesamte Breite der Lagen ineinandergewirkt werden, wobei jeweils in einer der beiden Lagen in einem Verbindungsbereich (6) zwischen den beiden Wirkschlauchteilen (4, 5) quer zur Längsrichtung der Wirkschlauchteile eine Trennstelle (7) vorgesehen ist und daß durch mittiges Trennen längs der genannten Naht- und Trennbereiche einzelne Halterungen fertiggestellt werden.

10. Verfahren nach Anspruch 9,
dadurch gekennzeichnet,
daß mit Abstand zu den Trennstellen (7) im ersten Wirkschlauchteil (4) jeweils weitere Trennstellen zum Festlegen an einer Kniescheibe des Benutzers erzeugt werden.

11. Verfahren zur Herstellung einer Halterung nach Anspruch 5,
dadurch gekennzeichnet,
daß Hosen (14, 15) fortlaufend und paarweise in zwei Lagen (11, 12) in doppelfonturiger Wirkweise quer zur Längsrichtung der Beinteile (26) gewirkt werden, indem in gleichmäßigen Abständen die beiden Lagen (11, 12) zur Bildung von Naht- und Trennbereichen (13, 19) ineinandergewirkt werden, wobei jeder zweite Naht- und Trennbereich (13) über die gesamte Breite der Lagen verläuft und jeder zwischen diesen liegende Naht- und Trennbereich (19) parallel dazu in gleichem Abstand von den ersteren nur einen mittleren Teilbereich symmetrisch zu den Seitenkanten der Lagen (11, 12) umfaßt und wobei von den Enden der letzteren Naht- und Trennbereiche (19) senkrecht zu den Beinteilen (26) verlaufende, bis zu ersteren Naht- und Trennbereichen (13) reichende deckungsgleiche Trennschlitze (20, 21) in beiden Lagen (11, 12) verlaufen, wobei jeweils in einer der Lagen (11) senkrecht zur Richtung der Beinteile (26) eine weitere kurze Trennstelle (17) vorgesehen ist und daß durch mittiges Trennen der genannten Naht- und Trennbereiche (13, 19) einzelne Hosen voneinander abgetrennt werden.

## Claims

1. Holding means for holding a urine bag at a leg, especially at the thigh (C), of a user, having a hose part which may be pulled over the leg,
characterised in
that the hose part consists of two parts, a first knitted hose part (4, 24) which may be pulled over the leg (C) and a second knitted hose part (5, 25) of the same diameter which is connected to said first hose part by a continuous connecting region (6, 16) and which, for the purpose of securing the urine bag (D), may be folded over upwardly from below onto the first knitted hose part (4, 24), and of a separating region (7, 17) in the connecting region (6, 16) to allow the passage of a drain (E) of the urine bag (D) when the knitted hose parts (4, 5, 24, 25) are folded over one another.

2. Holding means according to claim 1,
characterised in
that the first knitted hose part (4) comprises a knitted fabric with a higher percentage of elastic thread:

3. Holding means according to any one of claims 1 or 2,
characterised in
that, for the purpose of increasing the friction effect, the first knitted hose part (4) is produced by using rubber-coated threads.

4. Holding means according to any one of claims 1 to 3,
characterised in
that at least the first knitted hose part (4) comprises a slit for being fixed to the knee cap of the user when the urine bag is arranged at the lower leg of the user.

5. Holding means according to claim 1,
characterised in
that the first knitted hose part (24) forms the leg part (26) of a pair of trousers (14).

6. Holding means according to any one of claims 1 to 5,
characterised in
that the connecting region (6, 16) consists of loosely knitted threads.

7. Holding means according to any one of claims 1 to 6,
characterised in
that at least one marking thread in a contrasting colour is inserted into the connecting region (6, 16).

8. Holding means according to any one of claims 1 to 7,
characterised in
that a holding band (8) is provided at the free end of the second knitted hose part (5, 25).

9. A process of producing holding means according to claim 1,
characterised in
that the knitted hose parts (4, 5) are knitted continuously in two layers (1, 2) in a double section knitting operation transversely to the longitudinal direction of the knitted hose parts in that, for the purpose of forming seam and separating regions (3), the two layers, at equal distances, are knitted into one another over the entire width of the layers, that in one of the two layers, in a connecting region (6) between the two knitted hose parts (4, 5), there is provided a separating region transversely to the longitudinal direction of the knitted hose parts, and that individual sets of holding means are produced by separating them centrally along the said seam and separating regions.

10. A process according to claim 9,
characterised in
that at a distance from the separating regions (7), in the first knitted hose part (4), there are provided further separating regions for being attached to one of the user's knee caps.

11. A process of producing holding means according to claim 5,
characterised in
that trousers (14, 15) are knitted continuously and in pairs in two layers (11, 12) in a double section knitting manner transversely to the longitudinal direction of the leg parts (26) in that, for forming seam and separating regions (13, 19), the two layers (11, 12) are knitted into one another at equal distances, with each second seam and separating region (13) extending across the entire width of the layers and with each seam and separating region (19) positioned therebetween, in parallel thereto and at the same distance from the former, comprising only a central partial region positioned symmetrically relative to the side edges of the layers (11, 12) and with congruent separating slits (20, 21) extending in both layers (11, 12) from the ends of the latter seam and separating regions (19) perpendicularly to the leg parts (26) as far as the former seam and separating regions (13), with a further short separating region (17) being provided in one of the layers (11) perpendicularly to the direction of the leg parts (26) and that by centrally separating the seam and separating regions (13, 19) individual pairs of trousers are separated from one another.

## Revendications

1. Dispositif de maintien pour une poche urinaire (D) sur la jambe, en particulier sur la cuisse (C) d'un utilisateur, comprenant une partie de tuyau qui peut être erfilée sur la jambe, caractérisé par le fait que la partie de tuyau est formée de deux parties,
une première partie de tuyau tricoté (4, 24) qui peut être enfilée sur la jambe (C), et une seconde partie de tuyau tricoté (5, 25) de même diamètre qui est reliée à la première via une zone de raccordement périphérique (6, 16), et qui peut être rabattue du bas vers le haut sur la première partie de tuyau tricoté (4, 24) pour fixer la poche urinaire (D), et comportant un emplacement de séparation (7, 17) dans la zone de raccordement (6, 16) pour le passage d'un organe d'évacuation (5) de la poche urinaire (D) lorsque les deux parties de tuyau tricoté (4, 5, 24, 25) sont rabattues l'une sur l'autre.

2. Dispositif de maintien selon la revendication 1, caractérisé en ce que la première partie de tuyau tricoté (4) comporte un tissu à mailles avec une proportion élevée de fibres élastiques.

3. Dispositif de maintien selon l'une ou l'autre des revendications 1 et 2, caractérisé en ce que la première partie de tuyau tricoté (4) est réalisée en utilisant des fibres caoutchoutées afin d'augmenter l'effet de friction.

4. Dispositif de maintien selon l'une quelconque des revendications 1 à 3, caractérisé en ce que la première partie de tuyau tricoté au moins (4) comporte une fente pour la fixation sur la rotule de l'utilisateur lors de l'agencement de la poche urinaire sur la jambe inférieure de l'utilisateur.

5. Dispositif de maintien selon la revendication 1, caractérisé en ce que la première partie de tuyau tricoté (24) forme une jambe (26) d'une culotte ou pantalon (14).

6. Dispositif de maintien selon l'une quelconque des revendications 1 à 5, caractérisé en ce que la zone de raccordement (6, 16) est constituée de fibres tricotées de façon lâche.

7. Dispositif de maintien selon l'une quelconque des revendications 1 à 6, caractérisé en ce que dans la zone de raccordement (6, 16) est mis en place au moins un fil de marquage distinct quant à sa couleur.

8. Dispositif de maintien selon l'une quelconque des revendications 1 à 7, caractérisé en ce qu'il est prévu un lacet de maintien (8) à l'extrémité libre de la seconde partie de tuyau tricoté (5, 25).

9. Procédé pour la fabrication d'un dispositif de maintien selon la revendication 1, caractérisé en ce que les parties de tuyau tricoté (4, 5) sont tricotées en continu en deux couches (1, 2) suivant un mode de tricotage à double fonture transversalement à la direction longitudinale des parties de tuyau tricoté, en ce que l'on tricote l'une dans l'autre et à des distances régulières les deux couches afin de former des régions de couture et des régions de séparation (3) sur la largeur totale des couches, un emplacement de séparation (7) étant prévu respectivement dans l'une des deux couches dans une zone de raccordement (6) entre les deux parties de tuyau tricoté (4, 5) perpendiculairement à la direction longitudinale des parties de tuyau tricoté, et en ce que l'on réalise des dispositifs de maintien isolés en séparant au milieu le long desdites zones de couture et de séparation précitées.

10. Procédé selon la revendication 9, caractérisé en ce que l'on produit d'autres emplacements de séparation à distance des emplacements de séparation (7) dans la première partie de tuyau tricoté (4) pour la fixation sur une rotule de l'utilisateur.

11. Procédé pour la fabrication d'un dispositif de maintien selon la revendication 5, caractérisé en ce que l'on tricote en continu et par paire des culottes ou pantalons (14, 15) en deux couches (11, 12) suivant un mode de tricotage à double fonture transversalement à la direction longitudinale des parties de la jambe (26), en tricotant l'une dans l'autre et à des distances régulières, les deux couches (11, 12) pour former des zones de couture et des zones de séparation (13, 19) ; une zone de couture et de séparation sur deux (13) s'étendant sur la totalité de la largeur des couches, et chaque zone de couture et de séparation (19) située entre celles-ci comporte uniquement une région partielle centrale parallèlement à celle-ci et à la même distance de la première, symétriquement par rapport aux rives latérales des couches (11, 12), et depuis les extrémités des dernières zones de couture et de séparation (19), des fentes de séparation (20, 21) s'étendent dans les deux couches (11, 12) perpendiculairement aux parties (26) de la jambe, en s'étendant jusqu'aux premières zones de couture et de séparation (13), en étant de même étendue ; en ce que dans l'une des couches respectives (11) et perpendiculairement à la direction des parties (26) de la jambe est prévu un autre emplacement de séparation court (17) ; et en ce que l'on sépare des culottes ou des pantalons individuels les uns des autres en séparant au centre les zones de couture et de séparation précitées (13, 19).
